# EUROPEAN PATENT SPECIFICATION

(11) **EP 0 572 963 B1**
(45) Date of publication and mention of the grant of the patent: **20.09.2000**
(21) Application number: 93108735.7
(22) Date of filing: 31.05.1993
(51) Int. Cl.: A61K 31/60, A61K 47/10

(54) **Sclerosing solution**
Sklerosierende Lösung
Solution sclérosante

(30) Priority: 02.06.1992 IT GE920060
(43) Date of publication of application: 08.12.1993
(73) Proprietor: Capurro, Sergio, I-16121 Genova (IT)
(72) Inventor: Capurro, Sergio, I-16121 Genova (IT)
(74) Representative: Maritano Maello, Giovanna

(56) References cited:
- WO-A-90/09779
- US-A- 4 016 264
- PHLEBOLOGY vol. 4, no. 1 , March 1989 pages 51 - 54 CORCOS L. ET AL 'COMBINED LASER AND SCLEROTHERAPY FOR TELANGIECTASIS OF THE LOWER LIMBS'
- DATABASE WPI Section Ch, Week 8725, Derwent Publications Ltd., London, GB; Class A12, AN 87-174449 & JP-A-62 106 022 (NASA KK) 16 May 1987
- PHLEBOLOGIE vol. 3 , July 1966 pages 235 - 241 TOURNAY R. 'TRAITEMENT SCLEROSANT DES TRES FINES VARICISITES INTRA OU SOUS DERMIQUES'

## Description

This invention relates to a high efficacy sclerosing solution causing no iatrogenic lesions and process to obtain this solution, consisting of a sodium salicylate and glycerol water solution obtained by dissolving salicylic acid and sodium carbonate or sodium salicylate to reach a concentration ranging between 3% and 12% in weigth with glycerol in bidistilled water at a 10% to 60% concentration in weigth.

According to the prior art, thanks to their irritating action on the vasal endothelium, sclerosing solutions, if injected in a small quantity, for example, into veins, venules or into ectatic capillaries, cause coarctation of their lumen until they are no longer visible.

The main inconvenient caused by the known sclerosing solutions, like for example hydroxy-polyethoxy-dodecane and sodium tetradecyl-sulfate-based solutions, is that quite often their irritating action cannot be foreseen because of a different individual response to these solutions. A well balanced irritating action, instead, is very important because, if it is insufficient, the desired effect is not obtained, whereas, if the sclerosing solution is very aggressive to the endothelium, it will cause thrombosis of veins, venules and capillaries. This phenomenon must be avoided since it causes a non-esthetic effect which can be eliminated either by evacuating the thrombosed blood through puncture or microincision of the vessel or which disappears after a long time. When the sclerosing solution is very aggressive, it may even determine lesions and fissurations of the vessel wall, with a consequent outflow of blood pigments, causing non-esthetic post-sclerotherapeutic pigmentations difficult to resolve. If the sclerosing solution is too aggressive, skin necrosis may occur if said solution comes into contact with the dermis of with the subcutaneous tissue, this being due to rupture of the vessel during injection or to an operator's error. With the use of the known solutions, necrosis may occur, especially in the most distal regions of the lower limbs, even though the injection has been correctly made. The known hypertonic sodium chloride solutions, too, can frequently cause post-sclerotherapeutic pigmentations and necrosis.

Frequently, treatments with sclerosing solutions have esthetic purposes; this is why the use of sclerosing solutions potentially causing iatrogenic lesions, like necrosis and pigmentations, shall basically be avoided.

According to the prior art, to obviate these disadvantages, only sclerosing solutions were used not causing skin necrosis even if injected into the perivasal tissue; however, these sclerosing solutions are little efficacious, therefore, numerous injections into the same capillary are necessary, thus resulting in a considerable number of sclerotherapeutic applications.

As known from the Phlebologie journal, vol. 3 (1966) pages 235-241 for "intra or hypodermal sclerosing treatments of very thin varicosities" either a solution consisting of 10, 12, 15% sodium salicylate concentration alone can be employed or a solution consisting of bidistilled glycerol with chrome alum (= trivalent chrome), called chromic glycerine. These sclerosing solutions, however, present further disadvantages.

Chromic glycerine has a high trivalent chrome content, to which some Authors ascribe a mutagen activity and, however pure the chrome salt may be, it still always contains a certain amount of heavy metals harmful for the organism and which, sometimes, cause local or general allergic phenomena or other severe reactions like cardiac pain or deliquium. Furthermore, if a high quantity of chromic glycerin is injected, the irritating action induced by chrome can cause renal injury and microhematuria; consequently, chromic glycerin cannot be injected in the appropriate quantity as it should often be required for patient's rapid treatment. Chromic glycerin injection can also produce a sensation of local burning.

Sodium salicylate is a little efficacious sclerosing solution, it is normally considered as a weak sclerosing solution. Injecting this solution at the conventional concentration rates used according to the known technique, i.e. from 12% to 30%, is very painful.

In patent WO-A-90/09 779, inventor Benhuri, a "Composition for treatment of periodontal disease" is described. It is a mouth wash containing sodium salicylate, chlorexidine gluconate and allantoin. The inventor plans to add also glycerine which provides the composition with the desired feel and texture and is an aqueous carrier. However, said composition can be employed only externally (for topical use), and it is not designed for being injected, nor could it be injected, because chlorexidine gluconate is toxic if injected into a vein. Therefore, it cannot work as a sclerosing agent, which is the object and the aim of this invention.

In patent US-A-4 016 264 inventor Clark, a "Wart treatment with phosphonoacetic acid or derivatives thereof' is described. In the examples mentioned under no. 3 and 4, glycerine and salicylic acid (not salycilated sodium as in this invention) are jointly employed, but salycilic acid if injected could cause severe consequences. In any case, salicylic acid (5-10%) and glycerine (5%) doses are not suitable to cause sclerosis. Furthermore, the product contains also and above all phosphonoacetic acid, which by no means can be injected into veins, because phosphonoacetic acid is of caustic nature. Therefore, no sclerosing function - the object and aim of this invention - can be achieved even with this product.

Object of this invention is to realize a sclerosing solution having a greater efficacy than the known sclerosing solutions and the injection of which is virtually painless, not producing local iatrogenic injury, like thrombosis, post-sclerotherapeutic pigmentations and skin necrosis, without any local reactions like redness, oedema, itching or postoperative pain, nor general reactions, i.e. a sclerosing solution causing no troubles when injected at a dose capable of sclerosing a high number of ectatic vessels.

For this purpose, a sclerosing solution has been realized - hereinafter currently called "salicylic glycerin" - obtained by dissolving sodium salicylate and glycerol in water. This solution has been then brought to a physiologic pH value with a buffer system.

All numerous expected advantages can be obtained with salicylic glycerin: salicylic glycerin is more efficacious than the above-mentioned known solutions even though its active constituents, i.e. glycerin and sodium salicylate, are combined at a much lower concentration rate than it occurs for the individual constituents in the known solutions; it is well tolerated by the organism even if injected in massive quantities; it has an excellent local tolerance; the treatment area does not become edematous, it causes no iatrogenic damage even when injected into the perivasal tissue, nor any general reactions. A salicylic glycerin injection is almost painless, though enough for the patient to warn the operator when he is erroneously injecting into the perivasal tissue.

As compared with chromic glycerin, salicylic glycerin offers the dual advantage of having eliminated the harmful trivalent chrome salts, being replaced with a low sodium salicylate content, and of having a much prompter action.

Sodium salicylate is extremely well tolerated, no allergies have ever been observed when injected into the vein. Indeed, sodium salicylate solutions are even administered by phleboclysis in certain therapies, just like glycerin solutions are injected by phleboclysis.

This invention has been realized based on the observation that a solution at a low sodium salicylate concentration rate - say 6% - was found to be not appropriate to sclerose ectatic capillaries, although it was repeatedly injected into the same vessel. However, an immediate inflammatory reaction of the endothelium of the vessel was observed, which appeared to be reddened along its path.

Based on this observation, it was assumed that trivalent chrome in a chromic glycerin solution has an irritant action similar to that obtained at low sodium salicylate concentration rates, though by far less efficacious and involving the above-mentioned risks caused by chrome and by the impurities of its salt.

It was proved that this assumption was correct and that glycerin associated with low sodium salicylate concentration rates produces a much quicker sclerosis that the already known chromic glycerin and sodium salicylate solutions. In other words, there is a sum up action.

Unlike the use prescribed for other sclerosing solutions, salicylic glycerin solutions having a higher sodium salicylate content are used for smaller vessels, whereas lower concentrations are used for veins and venules.

Therefore, a larger quantity of solution can be injected, which allows to treat an extensive area.

According to this invention, the natural pH value of salicylic glycerin is modified during its preparation in order to equal the human physiologic pH value through a buffer system, based, for example, on bibasic sodium phosphate or on sodium citrate. It was noticed, in fact, that the pain that the patient feels during injection of sodium salicylate solution and of chromic glycerin solution is mainly due to the non-physiologic pH value of these solutions.

According to this invention, the percentage of sodium salicylate contained in salicylic glycerin is ranging from 3% to 12% in weigth, whereas the glycerol percentage varies from 10% to 60% in weigth.

For illustrative purposes, some examples of salicylic glycerin preparations, according to this invention, are reported here below.

### 1st EXAMPLE - Sclerosing solution according to this invention having a 6% sodium salicylate concentration.

Preparation of 500 ml of solution: 30 g of sodium salicylate F.U. (Official Pharmacopoeia) have been weighed and dissolved in 150 ml of water.

Glycerol has been added at the rate of 175 g (for the 35% conc. glycerol preparation) or of 150 g (for the 30% conc. glycerol preparation). To modify the pH value of salicylic glycerin through a buffer system, 6 g of dodecahydrate bibasic sodium phosphate have been dissolved separately in 200 ml of water. This solution has been added to the previous one, having a pH value = 8.7. Then, the pH value has been brought to 7.5 with 10% conc. phosphoric acid. The product has been quantitatively transferred into a 500-ml volumetric flask and the solution brought to volume with bidistilled water. After sterile filtration through a sterilizing membrane, for ex.0.45 µ, the solution has been distributed into yellow actinic glass phials.

Sterility of the product may be assured either by autoclaving or with the use of sterile equipment in an aseptic environment.

### 2nd EXAMPLE - Sclerosing solution according to this invention having a 20% glycerol concentration and a buffer effect obtained through a sodium citrate F.U./citric acid F.U. system. The 11% conc. sodium salicylate is prepared extemporaneously from salicylic acid and from sodium carbonate.

Preparation of 10 litres of salicylic glycerin solution: 0.950 kg of salicylic acid F.U. have been dissolved in 7 litres of water. 0.426 kg of sodium carbonate monohydrate F.U. have been added and the mixture and stirred to complete dissolution. 2 kg of glycerol F.U. have been added. Separately, 0.056 kg of sodium citrate F.U. and 0.002 kg of citric acid F.U. have been dissolved in 1 litre of filtered bidistilled water. This solution has been added to the previous one. The pH value has been brought to 7.5 with q.s. of sodium carbonate or of citric acid. The product has been quantitatively transferred into a 10-l volumetric flask, the solution diluted to this volume with filtered bidistilled water.

Then, the solution has been filtered through a 0.2 µ membrane in an aseptic environment, then distributed, under sterile conditions, into yellow actinic glass phials.

Sterile equipment and an aseptic environment are necessary or else the solution has to be autoclaved at 121°C for 30 minutes.

## Claims

1. Sclerosing solution, characterized in that the solution consists of a sodium salicylate and glycerol water solution, wherein the sodium salicylate content is ranging from 3% to 12% in weigth and that the glycerol content varies from 10% to 60% in weigth and wherein the natural pH value of the solution is modified through a buffer system during the preparation phase, to equal the human physiologic pH value.

2. Sclerosing solution, as defined in claim 1, characterized in that it was obtained by dissolving 30 g of sodium salicylate in 150 ml of water, with the addition of glycerol at a rate of 175 g (for the 35% glycerol solution concentration), or 150 g (for the 30% glycerol preparation concentration), having separately dissolved 6 g of dodecahydrate bibasic sodium phosphate in 200 ml of water, then adding this solution to the previous one having a pH value = 8.7, and then bringing the pH value to 7.5 with 10% phosphoric acid concentration, transferring it quantitatively into a 500-ml volumetric flask, that the solution was brought to volume with bidistilled water and that after filtering it through a sterilizing membrane in an aseptic environment the solution was distributed into yellow actinic glass phials.

3. Sclerosing solution, as defined in claim 1, characterized in that it has a 20% glycerol concentration and that the buffer effect is obtained through a sodium citrate/citric acid system, having a 11% sodium salicylate concentration rate, prepared extemporaneously from salicylic acid and from sodium carbonate, having used 0.950 kg of salicylic acid in 7 litres of water to prepare 10 litres of solution, after adding 0.426 kg of sodium carbonate monohydrate and stirring until completely dissolved, having added 2 kg of glycerol, having separately dissolved 0.056 kg of sodium citrate and 0.002 kg of citric acid in 1 litre of filtered bidistilled water and having added this solution to the previous one, then bringing the pH value to 7.5 with q.s. of sodium carbonate or of citric acid, then transferring the product quantitatively into a 10-l volumetric flask and diluting the solution to this volume with filtered bidistilled water, and that after filtering through a 0.2 µ membrane in an aseptic environment the solution was distributed, under sterile conditions, into yellow actinic glass phials.

4. Process to obtain the sclerosing solution, as defined in claims 1 and 4, wherein 500 ml of solution were prepared by dissolving 30 g of sodium salicylate in 150 ml of water, with the addition of glycerol at a rate of 175 g (for the 35% glycerol solution concentration), or 150 g (for the 30% glycerol preparation concentration), having separately dissolved 6 g of dodecahydrate bibasic sodium phosphate in 200 ml of water, then adding this solution to the previous one having an 8.7 pH value, and then bringing the pH value to 7.5 with 10% phosphoric acid concentration (1.5 ml), transferring it quantitatively into a 500-ml volumetric flask, that the solution was brought to volume with bidistilled water and that after filtering it through a sterilizing membrane in an aseptic environment the solution was distributed into yellow actinic glass phials.

5. Process to obtain the sclerosing solution, as defined in claims 1 and 5, characterized in that the solution has a 20% glycerol concentration and that the buffer effect is obtained through a sodium citrate/citric acid system, having a 11% sodium salicylate concentration rate, prepared extemporaneously from salicylic acid and from sodium carbonate, having suspended 0.950 kg of salicylic acid in 7 litres of water to prepare 10 litres of solution, after adding 0.426 kg of sodium carbonate monohydrate and stirring until completely dissolved, then adding 2 kg of glycerol, having separately dissolved 0.056 kg of sodium citrate and 0.002 kg of citric acid in 1 litre of filtered bidistilled water and having added this solution to the previous one, then bringing the pH value to 7.5 with q.s. of sodium carbonate or of citric acid, then transferring the product quantitatively into a 10-l volumetric flask and diluting the solution to this volume with filtered bidistilled water, and that after filtering through a 0.2 µ membrane in an aseptic environment the solution was distributed, under sterile conditions, into yellow actinic glass phials.

6. Process to obtain the sclerosing solution, as defined in claim 1, wherein the solution shall be prepared with the use of sterile equipment in an aseptic environment or be subsequently autoclaved at 121°C for 30 minutes.

## Patentansprüche

1. Sklerosierende Lösung, dadurch gekennzeichnet, daß die Lösung aus Natriumsalicylat und wässriger Glyzerollösung besteht, wobei der Natriumsalicylatanteil von 3% bis 12% des Gewichtes beträgt und der Glyzerolanteil zwischen 10% und 60% des Gewichtes variiert und wobei der natürliche pH Wert der Lösung durch ein Puffersystem während der Zubereitung modifiziert wird, um sich den menschlichen pH Werten anzugleichen.

2. Sklerosierende Lösung,wie unter Anspruch 1 definiert, dadurch gekennzeichnet, daß sie durch Auflösung von 30 g Natriumsalicylat in 150 ml Wasser mit dem Zusatz von Glyzerol zu einem Anteil von 175 g (für die 35% Glyzerollösungskonzentration) oder 150 g (für die 30% Glyzerolpräparatkonzentration) erhalten wird, nachdem man getrennt 6 g dibasisches Natriumphosphat-Dodecahydrat in 200 ml Wasser aufgelöst hat, diese Lösung wird der ersteren Lösung beigefügt bei einem pH Wert von 8.7, danach wird durch Zusatz einer 10% Phosphorsäurekonzentration der pH Wert auf 7.5 gebracht indem die Lösung quantitativ in einen 500 ml Meßkolben überführt und durch bidestilliertes Wasser auf das Volumen gebracht wird, nachdem die Lösung durch eine sterilisierende Membran in einer aseptischen Umgebung gefiltert wurde, wurde sie schließlich in gelbe aktinische Glasampullen verteilt.

3. Sklerosierende Lösung, wie unter Anspruch 1 definiert, dadurch gekennzeichnet, daß sie eine 20% Glyzerolkonzentration hat und daß die Pufferwirkung durch ein Natriumzitrat/ Zitronensäuresystem erhalten wird, der Anteil an Natriumsalicylat beträgt 11% , das frisch aus Salicylsäure und Natriumcarbonat zubereitet wird, indem man 0.950 kg Salicylsäure in 7 Liter Wasser zur Zubereitung von 10 Liter Lösung verwendet, nachdem man 0,426 kg Natriumcarbonat-monohydrat unter ständigem Rühren bis zur völligen Auflösung zugefügt hat, es werden 2 kg Glyzerol hinzugefügt, getrennt werden 0.056 kg Natriumzitrat und 0.002 kg Zitronensäure in 1 Liter flitriertem bidestilliertem Wasser aufgelöst und diese Lösung wird der ersteren beigefügt, dann wird der pH Wert auf 7.5 gebracht durch q.s. von Natriumcarbonat oder Zitronensäure, dann wird das Präparat quantitativ in einen 10 l Meßkolben überführt und die Lösung zu diesem Volumen mit gefiltertem bidestilliertem Wasser verdünnt und nach Filtern der Lösung durch eine 0.2 µ Membran in einer aseptischen Umgebung wird die Lösung unter sterilen Bedingungen in gelbe aktinische Glasampullen verteilt.

4. Verfahren um die sklerosierende Lösung zu erhalten, wie unter Anspruch 1 und 4 definiert, wobei 500 ml der Lösung durch Auflösung von 30 g Natriumsalicylat in 150 ml Wasser, unter Zugabe von Glyzerol zu einem Anteil von 175 g (für die 35% Glyzerollösungskonzentration), oder 150 g (für die 30% Glyzerolpräparatkonzentration) zubereitet wird, indem man getrennt 6 g von dibasischem Natriumphosphat Dodecahydrat in 200 ml Wasser aufgelöst hat, und dann diese Lösung der ersteren bei einem pH Wert von 8,7 beifügt und dann den ph Wert durch Zugabe von 10% Phosphorsäure (1.5 ml) auf 7.5 bringt und die Lösung quantitativ in einen 500 ml Meßkolben bringt, die Lösung wird durch bidestilliertes Wasser auf das entsprechende Volumen gebracht und nach Filtern durch eine sterilisierende Membran in aseptischer Umgebung wird die Lösung in gelbe aktinische Glasampullen verteilt.

5. Verfahren um die sklerosierende Lösung zu erhalten, wie unter Anspruch 1 und 5 definiert, dadurch gekennzeichnet, daß die Lösung eine 20% Glyzerolkonzentration besitzt und daß die Pufferwirkung durch ein Natriumzitrat/ Zitronensäuresystem erhalten wird, man hat eine 11% Natriumsalicylatkonzentration, die frisch aus Salicylsäure und Natriumcarbonat hergestellt wird, indem man 0.950 kg Salicylsäure in 7 Liter Wasser zur Zubereitung von 10 Liter Lösung verwendet, nachdem man 0,426 kg Natriumcarbonat-monohydrat unter ständigem Rühren bis zur völligen Auflösung zugefügt hat, es werden 2 kg Glyzerol hinzugefügt, getrennt werden 0.056 kg Natriumzitrat und 0.002 kg Zitronensäure in 1 Liter flltriertem bidestilliertem Wasser aufgelöst und diese Lösung wird der ersteren beigefügt,dann wird der pH Wert auf 7.5 gebracht durch q.s. von Natriumcarbonat oder Zitronensäure, dann wird das Präparat quantitativ in einen 10 l Meßkolben überführt und die Lösung zu diesem Volumen mit gefiltertem bidestilliertem Wasser verdünnt und nach Filtern der Lösung durch eine 0.2 µ Membran in einer aseptischen Umgebung wird die Lösung unter sterilen Bedingungen in gelbe aktinische Glasampullen verteilt.

6. Verfahren, um die sklerosierende Lösung zu erhalten, wie unter Anspruch 1 definiert, wobei die Lösung unter Anwendung von sterilen Geräten in aseptischer Umgebung oder durch nachfolgende Sterilisierung im Autoklav bei 121° C für die Dauer von 30 Minuten zubereitet wird.

## Revendications

1. Solution sclérosante ***caractérisée*** par le fait que la solution est formée d'une solution en eau de salicylate de sodium et de glycérol ayant une teneur pondérale de 3% à 12% de salicylate de sodium et de 10% à 60% de glycerol et dans laquelle la valeur du pH naturel de la solution est modifiée par un système tampon au cours du processus de préparation pour atteindre la valeur pH physiologique humaine.

2. Solution sclérosante comme définie dans la revendication 1, ***caractérisée*** par le fait qu'elle a été obtenue en dissolvant 30 g de salicylate de sodium dans 150 ml d'eau, en ajoutant du glycerol dans la mesure de 175 g (pour obtenir une solution avec une concentration de 35% de glycerol ) ou de 150 g (pour préparer une solution ayant une concentration de 30% de glycerol), ayant séparément dissous 6 g de phosphate de sodium bibasique dodécahydré dans 200 ml d'eau, et qu'ensuite cette solution à été ajoutée à la précédente ayant une valeur de pH = 8,7, et que cette valeur de pH a été ensuite portée à 7,5 avec 10% de concentration d'acide phosphorique et le tout a été transféré dans un ballon jaugé dc 500 ml, et que la solution a été portée à volume avec de l'eau bidistillée et que la solution, filtrée à travers une membrane stérilisante dans un milieu aseptique, a été ensuite distribuée dans des fioles jaunes en verre actinique.

3. Solution sclérosante comme définie dans la revendication 1, ***caractérisée*** par le fait qu'elle a une concentration de 20% de glycérol et que l'effet tampon a été obtenu par un système de citrate de sodium/acide citrique, ayant une concentration de 11% de salicylate de soude, préparé en extemporanée avec de l'acide salicylique et du carbonate de soude, en utilisant 0,950 kg d'acide salicylique dans 7 litres d'eau pour préparer 10 litres de solution, et que 0,426 kg de monohydrate de carbonate de sodium ont été ensuite ajoutés et qu'à ce mélange, après avoir été agité jusqu'à sa dissolution, ont été ensuite ajoutés 2 kg de glycerol, ayant séparément dissous 0,056 kg de citrate de sodium et 0,002 kg d'acide citrique dans 1 litre d'eau bidistillée filtrée, et que cette solution a été ajoutée à la précédente et portée à la valeur de pH=7,5 avec une quantité suffisante de carbonate de sodium ou de l'acide citrique, avant de transférer ce produit quantitativement dans un ballon jaugé de 10 litres et ayant dilué la solution à ce volume avec de l'eau bidistillée et que cette solution, après filtrage à travers une membrane de 0,2 µ dans un milieu aseptique, a été distribuée en condition stérile dans des fioles en verre jaune actinique.

4. Processus pour obtenir la solution sclérosante comme définie dans les revendications 1 et 4 ***caractérisé*** par le fait que 500 ml de solution ont été préparés par dissolution de 30 g de salicylate de sodium dans 150 ml d'eau, en ajoutant du glycerol dans la mesure du 175 g (pour obtenir une solution avec une concentration de 35% de glycrol) ou 150 g (pour la solution ayant 30% de concentration en glycerol), ayant séparément dissous 6 g de phosphate de sodium bibasique dodécahydré dans 200 ml d'eau, et que cette solution a été ajoutée à la précédente ayant une valeur de pH = 8.7 et que cette valeur de pH a été portée à 7,5 avec 10% de concentration d'acide phosphorique (1,5 ml), et que le tout a été transféré dans un ballon jaugé de 500 ml et porté à volume avec de l'eau bidistiliée, et que la solution, filtrée à travers une membrane stérilisante dans un milieu aseptique, a été ensuite distribuée dans des fioles jaunes en verre actinique.

5. Processus pour obtenir la solution sclérosante comme défini dans les revendications 1 et 5, ***caractérisé*** par le fait que la solution a une concentration de 20% de glycérol et que l'effet tampon a été obtenu par un système de citrate de sodium/acide citrique ayant une concentration de 11% de salicylate de soucie, préparé en extemporanée avec de l'acide salicylique et du carbonate de soude, en utilisant 0,950 kg d'acide salicylique et 7 litres d'eau pour préparer 10 litres de solution, et que 0,426 kg de monohydrate de carbonate de sodium ont été ensuite ajoutés et qu'à ce mélange, après avoir été agité jusqu'à sa dissolution, ont été ensuite ajoutés 2 kg de glycerol, ayant séparément dissous 0,056 kg de citrate de sodium et 0,002 kg d'acide citrique dans 1 litre d'eau bidistillée filtrée, et que cette solution a été ajoutée à la précédente et portée à la valeur de pH=7,5 avec une quantité suffisante de carbonate de sodium ou de l'acide citrique, avant de transférer ce produit quantitativement dans un ballon jaugé de 10 litres et ayant dilué la solution à ce volume avec de l'eau bidistillée filtrée et que cette solution, après filtrage à travers une membrane de 0,2 µ dans un milieu aseptique, a été distribuée en condition stérile dans des fioles en verre jaune actinique.

6. Processus pour obtenir une solution sclérosante comme définie dans la revendication 1, ***caractérisé*** par le fait que la solution doit être préparée avec un appareillage stérile dans un milieu aseptique ou devra être successivement stérilisée à l'autoclave à 121°C pendant 30 minutes.
